# EUROPÄISCHE PATENTANMELDUNG

(11) **EP 1 180 721 A2**
(43) Veröffentlichungstag der Anmeldung: **20.02.2002**
(21) Anmeldenummer: 01117375.4
(22) Anmeldetag: 18.07.2001
(51) Int. Cl.: G03B 42/02, G03B 42/04, A61B 6/14

(54) **Filmhalter für die Anfertigung von Messaufnahmen bei angelegtem Kofferdamrahmen in der Paralleltechnik**

(30) Priorität: 28.07.2000 DE 10037677
(71) Anmelder: Stoica, Elisabeta-Cristina, 76297 Stutensee (DE)
(72) Erfinder: Stoica, Elisabeta-Cristina, 76297 Stutensee (DE); Ahlers, M. Oliver, Dr.med.dent., 20249 Hamburg (DE)
(74) Vertreter: Eisenführ, Speiser & Partner

(57) **Zusammenfassung**

Aus Gründen der Infektionsprophylaxe, der Asepsis und der Forensik ist die Kofferdamanwendung in der Endodontie indiziert. Die Anwesenheit von Kofferdamklammern, -gummi, -rahmen und Wurzelkanalinstrumenten macht die Positionierung herkömmlicher speziell für Meßaufnahmen entwickelten Filmhalter aufgrund ihres Aufbaus unmöglich: die Filmklemme (400) ist an dem Aufbißblock (100) befestigt, auf den der Patient beißt, um den Filmhalter während der Meßaufnahme intraoral zu fixieren. Solche Filmhalter müssen vor der Außenseite des Kofferdams positioniert werden.
Durch einen anderen Aufbau ermöglicht der neue Filmhalter die Anfertigung von Meßaufnahmen bei angelegtem Kofferdamrahmen: die Zähne der nicht zu behandelnden Seite fixieren den Aufbißblock (100), der durch ein Verbindungsstück (200) mit der Filmklemme (400) verbunden ist, die an dem zu behandelnden Zahn positioniert wird. Das aufgespannte Kofferdamgummi wird somit nur zur Seite geschoben und der neue Filmhalter von der Innenseite des Kofferdams positioniert. Dadurch wird jede Berührung des Filmhalters mit dem Zahn verhindert und das Behandlungsfeld bleibt trocken. Eine Zentriervorrichtung für die Anwendung der Paralleltechnik ist vorhanden.

## Beschreibung

Diese Erfindung bezieht sich auf einen Filmhalter für die Anfertigung von Meßaufnahmen während der endodontischen Behandlung bei angelegtem Kofferdamrahmen, bestehend aus: 1. einem trapezförmigen Aufbißblock, der durch mehrere von distal (hinten) nach mesial (vorne) durchgehenden und waagerecht ineinander übergehenden Perforationen, die je nach Kiefer und Kieferbreite die Verschiebung eines Verbindungsstückes ermöglichen, durchzogen ist, 2. zwei Verbindungsstücken für die Seiten- und die Frontzähne, die beide auf der einen Seite mit dem Aufbißblock und auf der anderen Seite mit der Filmklemme verbunden sind. An der Filmklemmezugewandten Seite befindet sich bei beiden Verbindungsstücken ein Zahnrad, das durch Umstecken der Filmklemme unterschiedliche Neigungen der Filmebene ermöglicht. An der Aufbißblock-zugewandten Seite befinden sich zwei runde parallel zueinander verlaufenden Stifte, die die rotationssichere Fixierung am Aufbißblock ermöglichen. Das Verbindungsstück für die Seitenzähne weist einen Knick auf, der die verbesserte Anpassung der Filmebene an die Seitenzähne ermöglicht. Das Verbindungsstück für die Frontzähne hat dagegen einen geradlinigen Verlauf und ist kürzer als das Verbindungsstück für die Seitenzähne. 3. zwei Filmklemmen: eine für die senkrechte und eine für die waagerechte Positionierung des Zahnfilmes. Die Filmklemme weist an ihrer Unterseite zwei Steckhalterungen mit unterschiedlichen Querschnittsformen auf: eine für das Zahnrad des Verbindungsstückes und eine rechteckige für den kurzen Schenkel des Indikatorstabes der Zentriervorrichtung. 4) einer Zentriervorrichtung bestehend aus einem L-förmigen Indikatorstab, der rechtwinklig von der Filmklemme (intraoral) nach extraoral verläuft, einem Zentrierring für die senkrechte Ausrichtung des Langtubus der Röntgenröhre bzw. des Zentralstrahls auf die Filmebene und einer Rechteckblende, die am Visierring angebracht wird und in der Mitte eine Aussparung von der Größe eines handelsüblichen Zahnfilmes hat.

Diese Erfindung bezieht sich auch auf einen Filmhalter, im folgenden teilweise auch Zahnfilmhalter genannt, für die Anfertigung von Meßaufnahmen während der endodontischen Behandlung mit oder ohne angelegtem Kofferdamrahmen, bestehend aus 1) einem rechteckigen Aufbißblock, 2) einem langen Verbindungsstück, dass sich in der Verlängerung des Aufbißblocks befindet, 3) einem kurzen Verbindungsstück, dass am Aufbißblock, bevorzugt senkrecht zu diesem , angebracht ist, 4) 2 Filmklemmen für die waagerechte (Längspositionierung) und HochkantPositionierung des Zahnfilms, die am kurzen Verbindungsstück aufsteckbar sind, 5) einer Zentriervorrichtung für den Röntgentubus, die am langen Verbindungsstück angebracht ist und schließlich 6) einer Rechteckblende, die in zwei Positionen in den Visierring aufsteckbar ist. Der Zahnfilmhalter kann in dieser Ausführungsform für Meßaufnahmen in der gesamten Mundhöhle eingesetzt werden. Dies wird durch die mehreren Positionen am kurzen Verbindungsstück des Zahnfilmhalters fixierbaren Filmklemmen und durch die wahlweise um 180 Grad verdrehte Positionierung des gesamten Zahnfilmhalters in der Mundhöhle ermöglicht.

Bei der Entwicklung des vorliegenden Filmhalters wurden hohe Ansprüche in Zusammenhang mit Strahlenschutz, Patientenkomfort, Zeitersparnis im Rahmen der Behandlung und leichte Handhabung durch den Zahnmediziner beachtet und erfüllt. Diese Anforderungen konnten nur durch einen neuen, sich von den anderen Filmhaltern unterscheidbaren Filmhalter erfüllt werden. Das Besondere an diesem Filmhalter ist der Zusammenbau seiner Bestandteile.

Herkömmliche Filmhalter für Meßaufnahmen haben einen Aufbißblock, an dem die Filmklemme fest verbunden ist. Aus diesem Grund ist ihre Fixierung in Anwesenheit einer Kofferdamklammer durch den zu untersuchenden Zahn oder seinen mesialen (vorderen) Nachbarzahn schwierig. Diese Anordnung machte die Positionierung herkömmlicher Filmhalter nur von der zu untersuchenden Seite (links oder rechts) möglich und erst nach Entfernung des Kofferdamrahmens, weil das auf dem Kofferdamrahmen aufgespannte -gummi die Positionierung des Zahnfilms nach oral hin verhindert. Im Rahmen der Endodontie bereitet diese Anordnung jedoch Probleme, weil die Anforderungen, die an den Kofferdam gestellt werden durch die Entfernung des Kofferdamrahmens nicht mehr erfüllt werden können.

Die Kofferdamklammer fixiert das Kofferdamgummi in der Mundhöhle und isoliert somit den zu untersuchende Zahn. Das Kofferdamgummi ist auf einem -rahmen aufgespannt, um das Behandlungsfeld übersichtlicher zu gestalten. Der Kofferdam hat in der Endodontie folgende Aufgaben:
1) den Zahn gegen die Mundhöhle zu isolieren, um zu verhindern, daß noch mehr Bakterien aus der Mundhöhle in den Wurzelkanal und nach apikal (= zur Wurzelspitze hin) gelangen,
2) durch die Desinfektion des Kofferdamgummis ein möglichst aseptisches (= keimfreies) Behandlungsfeld zu ermöglichen,
3) den Patienten vor Schlucken oder Aspirieren von Wurzelkanalinstrumenten zu schützen,
4) den Patienten vor Spüllösungen (z. B. Natriumhypochlorid), Blut, Eiter oder Bakterien zu schützen,
5) die Weichteile des Patientenmundes fern von spitzen oder rotierenden Instrumenten zu halten,
6) den Behandler und seine Assistenz vor Bakterien und Viren aus der Mundhöhle des Patienten zu schützen.
Diese Argumente verdeutlichen die Wichtigkeit des schon lange existierenden aber nur selten benutzten Kofferdams.

Um einen lückenlosen aseptischen Behandlungsverlauf im Rahmen einer endodontischen Sitzung zu gestalten, ist es wichtig auch während der radiologischen Untersuchung diesen Schutz für Patienten und Behandler aufrechtzuerhalten.

Bei der Positionierung herkömmlicher Filmhalter in der Mundhöhle war entweder die Entfernung des Kofferdamrahmens notwendig oder die Abnahme des oberen bzw. des unteren Kofferdamgummianteils vom Kofferdamrahmen. Durch die Elastizität des anfangs aufgespannten Kofferdamgummis zog sich dieses jedoch immer zusammen und ermöglichte somit die Befeuchtung/ Kontamination des Behandungsfeldes mit Speichel. Die Kontamination mit Speichel oder Blut erforderte nach der radiologischen Untersuchung eine erneute Desinfektion des Behandlungsfeldes, die zeitaufwendig war. Für den Patienten erwies sich der Zustand nach Abnahme des Kofferdamrahmens auch als belastend, da entlang der bereits befeuchteten Kofferdamgummi-lnnenfläche der Speichel aus dem Mund fließen konnte. Im ungünstigsten Fall wirkte das Kofferdamgummi als Schlauch für den Abfluß des Speichels aus dem Mund.

Für die Positionierung des neuen Filmhalters ist die Abnahme des Kofferdamrahmens nicht mehr notwendig. Die Grundidee für die Entwicklung des neuen Filmhalters basiert auf seiner intraoralen Fixierung durch Aufbiß fern von dem zu röntgenden Zahn. Dies wird realisiert dadurch, daß die Filmklemme und der Aufbißblock nicht miteinander verbunden sind, sondern an entgegengesetzten Enden des Filmhalters angebracht sind. D. h., daß die Filmklemme an dem zu behandelten Zahn angebracht und der Aufbißblock durch die Zahnreihen der entgegengesetzten Seite, d. h. kontralateral fixiert werden kann. Durch die neue keilförmige Gestaltung des Aufbißblocks ist eine optimale Fixierung des Filmhalters durch den Aufbiß der Seitenzähne möglich, weil bei der Mundöffnungsbewegung die Kiefer ebenfalls keilförmig auseinander klaffen.

Bevor der Filmhalter positioniert wird, sollte die Neigung der Filmklemme der Zahnneigung durch Umstecken des Zahnrades extraoral angepaßt werden.

Auf dem gleichen Prinzip basiert eine weitere Ausführungsform der Erfindung, bei der die Fixierung des Zahnfilmhalters ebenfalls fern von dem zu behandelten Zahn stattfindet, jedoch nicht kontralateral. Fixiert wird nun der Zahnfilmhalter durch Aufbiß der Zähne der gleichen Kieferseite, auf der sich der zu röntgende Zahn befindet. Bei dieser Ausführungsform sind die Filmklemme und der Aufbißblock im fertigen Zustand nicht unmittelbar miteinander verbunden. Zwischen diesen beiden Komponenten befindet sich bei dieser Ausführungsform vielmehr ein kurzes Verbindungsstück, das bevorzugt senkrecht und unlösbar, am Aufbißblock fixiert ist, und an dem eine Steckverbindung für beide Filmklemmen angebracht ist. Das heißt, dass die Filmklemme mit dem Zahnfilm an dem zu behandelnden Zahn angebracht und der Aufbißblock durch die Zähne in nicht unmittelbarer Nähe des zu untersuchenden Zahnes fixierbar ist. So würden je nach Kiefergröße des Patienten bei Aufnahmen im Seitenzahnbereich die Prämolaren oder die Frontzähne den Aufbißblock fixieren, während bei Aufnahmen im Frontzahnbereich die Seitenzähne den Aufbißblock fixieren würden. Auf diese Weise wird auch bei aufgespanntem Kofferdamrahmen eine einfache Positionierung des Zahnfilmes möglich. Für diese Ausführungsform wird auch getrennt von der übrigen Erfindung Schutz beansprucht, da diese Ausführungsform auch ohne die lösbare Verbindung von Aufbißblock und Filmklemme realisierbar und vorteilhaft ist.

Wie bei herkömmlichen Filmhaltern erfolgt die Einstellung des neuen Filmhalters für den zu untersuchenden Zahn außerhalb der Mundhöhle. Zuerst wird die Filmklemme für die gewünschte Position für Ober- und Unterkiefer eingesteckt. Entsprechend der Filmklemmen-Position wird der Visierring so eingestellt, dass der Zentralstrahl senkrecht und mittig auf den Zahnfilm trifft.

Die Positionierung des Filmhalters erfolgt, indem der Behandler den Kofferdamrahmen und die darauf aufgespannte Kofferdamfolie zur Seite des zu behandelnden Zahnes schiebt, um sich Zugang zur Mundhöhle zu verschaffen. So wird der Kofferdamrahmen dank der Gummielastizität insgesamt zu einer Seite geschoben, ohne das Behandlungsfeld zu beeinträchtigen. Zwischen dem Mundwinkel der Seite auf der nicht behandelt wird und dem aufgespannten Gummi ist somit genug Platz, um den Filmhalter in der Mundhöhle anzubringen und zu positionieren. Es wird erst die Seite mit der Filmklemme positioniert. Danach wird der Aufbißblock zwischen den Zahnreihen der gegenüberliegenden Seite so angebracht, dass die Zahnlängsachsen des zu röntgenden Zahnes und die Zahnfilmebene parallel zueinander sind. Anschließend erfolgt die sichere intraorale Fixierung des Zahnfilmhalters durch Zusammenbeißen. An der Filmklemme ist die Zielvorrichtung anzubringen, um den Langtubus des Röntgengerätes zu positionieren. Während der Meßaufnahme kann der Patient auf den Aufbißblock aufbeißen ohne die Instrumente zu berühren, sodaß kurzzeitig die Entspannung der Kaumuskulatur erfolgt.

Alternativ wird erst die Seite mit der Filmklemme parallel zur Längsachse des zu behandelnden Zahnes positioniert, wobei anschließend das Zusammenbeißen auf den Aufbißblock und somit die Fixierung des neuen Filmhalters durch die Zähne der gleichen Kieferseite, auf der sich der zu behandelnde Zahn sich befindet, erfolgt. Dies ist insbesondere vorteilhaft realisierbar mit der Ausführungsform, bei der zwischen dem Aufbißblock und der Filmklemme ein kurzes Verbindungsstück angebracht ist, um die Filmklemme und die Zentriervorrichtung relativ zu der Achse aus Aufbißblock und Führungsschiene zu versetzen. Der Vorteil dieser Versetzung ist die Positionierung und die anschließende Fixierung des Zahnfilmhalters ohne den zu behandelnden Zahn oder das von der übrigen Mundhöhle isolierte Behandlungsfeld zu beeinträchtigen. Auch für die Ausführungsform wird die Zentriervorrichtung - ebenfalls vor der intraoralen Positionierung des Filmhalters - auf der Führungsschiene so eingestellt, dass auch hier der Zentralstrahl senkrecht und mittig auf den Zahnfilm erfolgt. Dies kann auch ohne eine lösbare Verbindung zwischen Filmklemme und Aufbißblock realisierbar werden.

Auf dieser Weise wird bei Anwendung des neuen Filmhalters das Behandlungsfeld nicht befeuchtet und der Patient durch den Speichelfluß aufgrund des aus dem Mund heraushängenden Kofferdamgummis nicht belästigt. Aus diesem Grund ist nach der Anfertigung der Aufnahme die erneute Desinfektion des Behandlungsfeldes nicht notwendig und die Behandlungszeit wird verkürzt.

Der neuen Filmhalter muß in Verbindung mit einem radioluzentem Kofferdamrahmen eingesetzt werden, damit die Qualität der Meßaufnahmen durch den evtl. im Strahlenfeld sich befindenden Kofferdamrahmen nicht beeinträchtigt wird.

Es wird dem Behandler überlassen, ob ein starrer oder aufklappbarer Kofferdamrahmen Anwendung findet.

Aufgrund der vorhandenen Zentriervorrichtung ist der Einsatz der Rechtwinkeltechnik - oder auch der Paralleltechnik - zur Darstellung der Zähne möglich. Die parallele Einstellung der Filmebene zur Zahnlängsachse erfolgt, wie bei allen anderen Filmhaltersystemen, durch die manuelle Positionierung des Filmhalters seitens des Behandlers. Die Zentriervorrichtung ermöglicht die senkrechte Einstellung der Röntgenröhre zur Filmebene und somit den Einfall des Zentralstrahls senkrecht und mittig auf den Film. Dadurch werden alle Anteile des Zahnes gleichmäßig dargestellt. Für die maßgetreue Darstellung der Zähne sind die Abstandsverhältnisse Fokus-Film-Ebene verantwortlich. Da dieses Filmhaltesystem in Verbindung mit der Langtubus-Methode verwendet wird, kann, falls die Einstellung eines kleinen Film-Objekt-Abstand aufgrund anatomischer Gegebenheiten nicht möglich ist z. B. wegen eines flachen Gaumens, der Fokus-Film-Abstand mit Hilfe des Indikatorstabes verlängert werden, sodaß eine vergrößerte Darstellung des Zahnes vermieden wird.

Durch die Langtubus-Methode in Verbindung mit einer Zentriervorrichtung kann die Strahlenbelastung für den Patienten im Vergleich zur Halbwinkeltechnik stark reduziert werden. Durch das Anbringen der vorgesehenen Rechteckblende am Visierring wird die Strahlenbelastung für den Patienten jedoch ohne eine Qualitätsverminderung der Aufnahmen minimiert. Die Rechteckblende kann sowohl für die waagerechte als auch für die senkrechte Lage des Zahnfilmes mit Hilfe der am Visierring vorhandenen Markierungen eingesetzt werden.

Insgesamt trägt der oben beschriebene Filmhalter zu einer geringeren Strahlenbelastung und einem erhöhten Komfort des Patienten, zu einer Zeitersparnis und einer Arbeitserleichterung des Behandlers bei, ohne die Qualität der Meßaufnahmen zu beeinträchtigen.

Eine bevorzugte Ausführungsform wird im folgenden erläutert:

### Ausführungsform 1:

Filmhalter für die Anfertigung von Meßaufnahmen mit der Paralleltechnik bei angelegtem Kofferdamrahmen, bestehend aus einem Aufbißblock, zwei durch eine Steckhalterung an dem Aufbißblock lösbar fixierten Verbindungsstücken für die Seiten- und die Frontzähne, zwei an dem Verbindungsstück ebenfalls mit einer Steckhalterung gehaltenen Filmklemmen für die senkrechte und waagerechte Positionierung des Zahnfilmes, einem durch eine Steckhalterung an der jeweiligen Filmklemme gehaltenen L-förmigen Indikatorstabes und einem auf dem Indikatorstab in Längsrichtung des langen Schenkels des Indikatorstabes verschiebaren Visierringes mit einer Halterung zur Fixierung des Langtubus eines zahnärztlichen Röntgengerätes am Filmhalter und mit einer Rechteckblende, die mit Hilfe der Markierungen am Visierring sowohl waagerecht als auch senkrecht angebracht werden kann, dadurch gekennzeichnet, daß die mesiale (vordere), distale (hintere), untere und obere Fläche des Aufbißblocks rechteckig sind, wobei die untere Fläche kleiner als die obere ist und die distale Fläche kleiner als die mesiale ist, und die bukkale und orale Flächen trapezförmig und gleich groß sind, wobei die distale Kante des Trapezes kürzer als die mesiale ist, daß der Aufbißblock von mehreren von distal nach mesial durchgehenden und ineinander übergehenden runden Perforationen durchgezogen ist, die von einer gemeinsamen in der Mitte der distalen Aufbißblockfläche sich befindlichen Eintrittsöffnung parallel zur oberen und zur unteren Aufbißblockfläche verlaufen und auf der mesialen Aufbißblockfläche münden, daß diese Perforationen je nach Kieferbreite und behandeltem Zahn das Umstecken eines der beiden Verbindungsstücke, entweder des Verbindungsstückes für die Seitenzähne oder für die Frontzähne, ermöglichen, um die Länge des Filmhalters der Kieferbreite anzupassen, daß das Hauptstück des Verbindungsstücks für die Seitenzähne eine Abknickung seiner Längsachse aufweist und mit dem so entstandenen Nebenteil einen spitzen Winkel bildet und am Ende des Nebenteils -an der aufbißblockfernen Seite- ein Zahnrad als Steckhalterung für die Fixierung der Filmklemme aufweist, daß das Verbindungsstück für die Frontzähne kürzer ist als das Verbindungsstück für die Seitenzähne, einen geradlinigen Verlauf und kein Nebenstück hat und ebenfalls am aufbißblockfernen Ende ein Zahnrad mit dem gleichen Modul und Durchmesser hat wie das des Verbindungsstücks für die Seitenzähne, daß die beiden Filmklemmen einen ähnlichen Aufbau haben und sie sich voneinander nur in der Breite und Länge unterscheiden, wobei die eine Filmklemme für die senkrechte und die andere für die waagerechte Positionierung des Zahnfilmes geeignet ist, daß die Filmklemmen ein rechteckiges Rückschild, dessen Maße kleiner als ein handelsüblicher Zahnfilm für Erwachsene sind, und zwei vor dem Rückschild seitlich sich befindliche schmalen Leisten für die Fixierung des Zahnfilmes in der zwischen dem Rückschild und den beiden Leisten sich befindlichen Filmauflagerille während den Meßaufnahmen haben, daß unterhalb des Rückschildes und der beiden Leisten sich eine rechteckige Steckhalterung für die Fixierung des kurzen Schenkels des Indikatorstabes und hinter dieser Steckhalterung und dem Rückschild sich eine Steckhalterung für die Fixierung des Zahnrades der benötigten Filmklemme befinden, daß die Filmauflagerille sich in der Höhe des waagerechten Zahnes des Zahnrades befindet, daß der Indikatorstab einen rechteckigen Querschnitt hat und einen L-förmigen Verlauf, wobei der kleine Schenkel an der Filmklemme in der rechteckigen Steckhalterung fixiert wird und der lange Schenkel zur Verschiebung des Visierringes mit Hilfe einer am Visierring angebrachten zum Querschnitt des Indikatorstabes passenden Halterung dient, daß der Visierring einen runden Querschnitt vom Durchmesser eines Langtubus aufweist, an dem eine Rechteckblende mit einer rechteckigen Aussparung von der Größe eines Zahnfilmes angebracht werden kann.

Vorteilhafte Ausstattungen des Filmhalters sind in den Unteransprüchen gekennzeichnet.

Ausführungsbeispiele für die Erfindung werden nachstehend anhand der Zeichnungen erläutert. Es zeigen:
Fig. 1 eine schaubildliche Ansicht des für die Anfertigung von Meßaufnahmen in der Paralleltechnik im Oberkiefer-Seitenzahnbereich zusammengesetzten Filmhalters,
Fig. 2 eine schaubildliche Ansicht des für die Anfertigung von Meßaufnahmen in der Paralleltechnik im Oberkiefer-Frontzahnbereich zusammengesetzten Filmhalters,
Fig. 3 bis Fig. 13 einzelne Bestandteile des Filmhaltersatzes.

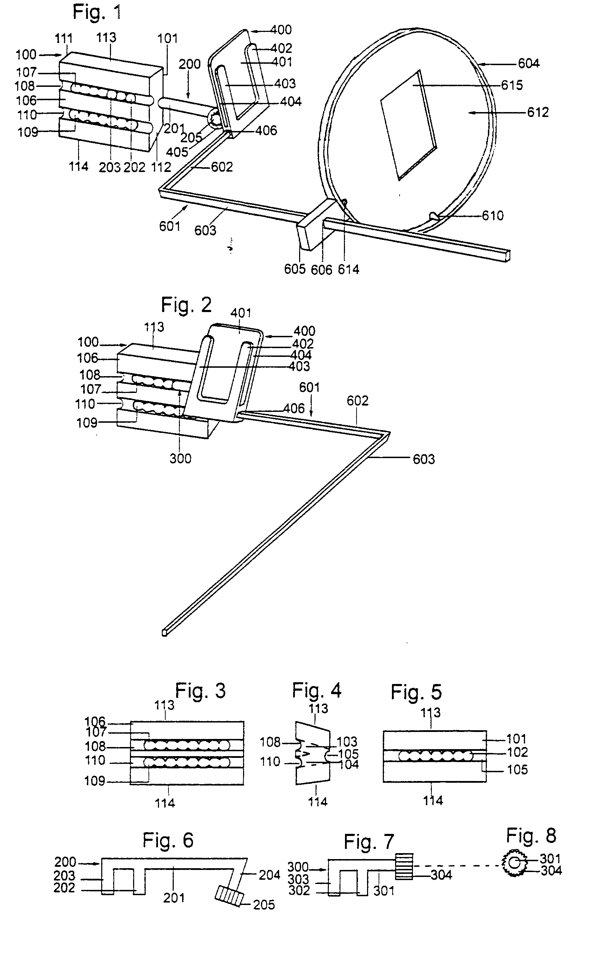

Fig. 3 eine mesiale Ansicht des Aufbißblocks 100 des Filmhalters,
Fig. 4 einen Schnitt in mesio-distaler Richtung durch die Mitte des Aufbißblocks 100 des Filmhalters,
Fig. 5 eine distale Ansicht auf den Aufbißblock 100 des Filmhalters,
Fig. 6 eine Ansicht von oben auf das Verbindungsstück 200 für die Seitenzähne,
Fig. 7 eine Ansicht von oben auf das Verbindungsstück 300 für die Frontzähne,
Fig. 8 einen frontalen Schnitt durch das Zahnrad der beiden Verbindungsstücke,

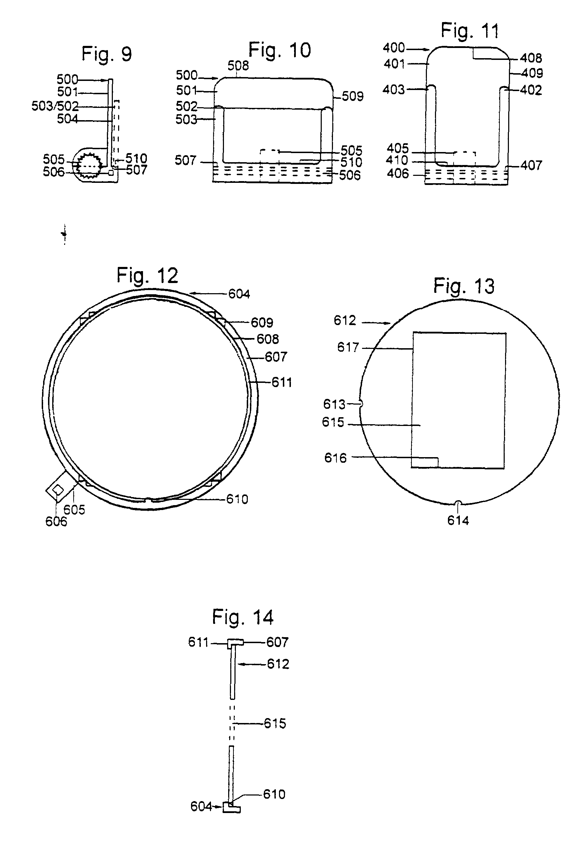

Fig. 9 einen Schnitt durch die Mitte der Filmklemme 500,
Fig. 10 eine Vorderansicht der Filmklemme 500 für die waagerechte Positionierung des Zahnfilmes,
Fig. 11 eine Vorderansicht der Filmklemme 400 für die senkrechte Positionierung des Zahnfilmes,
Fig. 12 eine Ansicht von der Röntgentubus-Seite auf den Visierring 604,
Fig. 13 eine Ansicht von der Röntgentubus-Seite auf die Rechteckblende 612,
Fig. 14 einen Schnitt durch den Visierring 604 bei eingesetzter Rechteckblende 612.

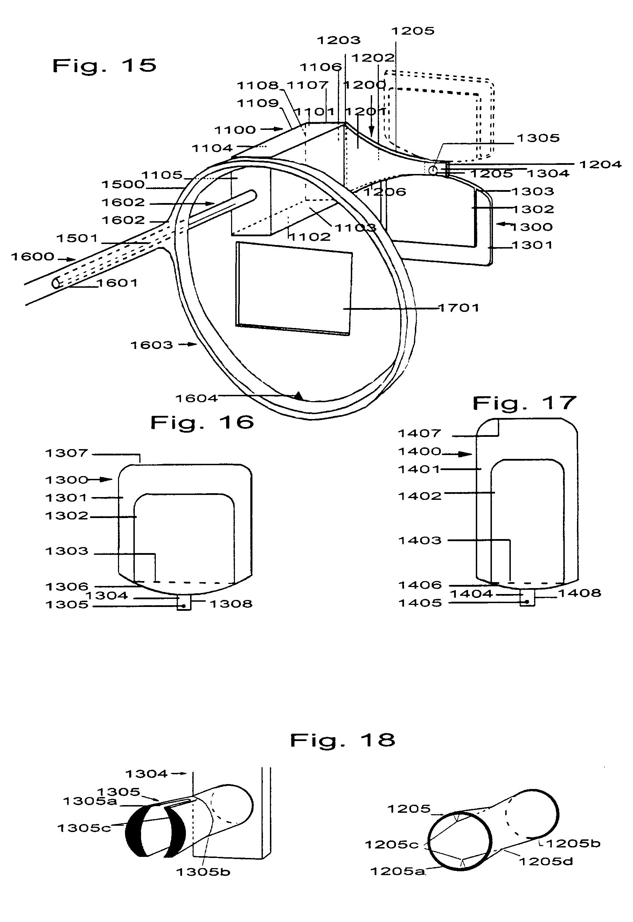

Fig. 15 eine schaubildliche Ansicht des neuen Zahnfilmhalters für die Anfertigung von Meßaufnahmen in der Paralleltechnik im dritten Quadranten, also auf der linken unteren Patientenseite. Die gestrichelten Linien zeigen die Positionierung der Filmklemme und des Visierringes bei der Anfertigung von Meßaufnahme im zweiten Quadranten bei einer Drehung der Filmklemme um ihre Rotationsachse um 180°,
Fig. 16 eine frontale Ansicht der Filmklemme 1300 für Aufnahmen bei waagerechter Positionierung des Zahnfilms,
Fig. 17 eine frontale Ansicht der Filmklemme 1400 für Aufnahmen im Frontzahnbereich bei Hochkantpositionierung des Zahnfilmes,
Fig.18 eine detaillierte Ansicht der Steckhalterung 1305 bzw. 1405 und der dazu passenden Öffnung 1205,

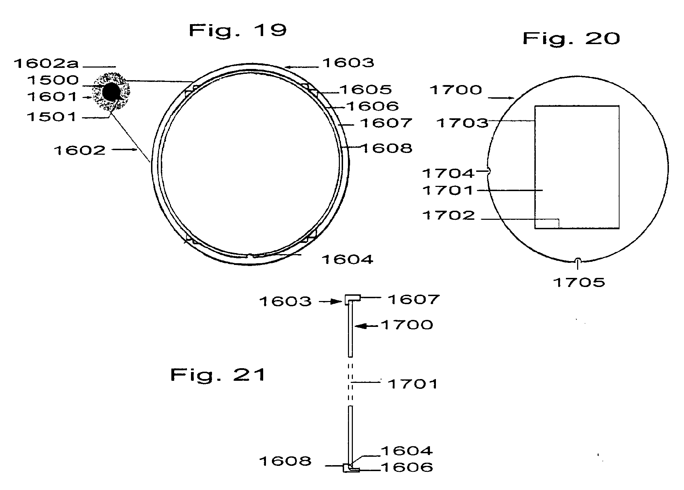

Fig. 19 eine schaubildliche Ansicht auf den Visierring 1603 und der Verbindung 1602,
Fig. 20 eine schaubildliche Ansicht auf die Rechteckblende 1700,
Fig. 21 einen Schnitt durch die im Visierring eingesetzte Rechteckblende 1700.

Fig. 1 zeigt eine schaubildliche Ansicht des neuen Filmhalters als Anwendungsbeispiel für die Durchführung von Meßaufnahmen in der Paralleltechnik im zweiten Quadranten (= Oberkiefer linke Patientenseite), wobei der Filmhalter durch den Aufbiß der Seitenzähne der rechten Patientenseite auf dem Aufbißblock in der gewünschten Position fixiert wird. Der dargestellte Instrumentensatz besteht aus einem Aufbißblock 100, einem durch eine Steckhalterung 103 mit dem Aufbißblock 100 lösbar verbundenen Verbindungsstück 200, einer mittels eines Zahnrads 205 mit dem Verbindungsstück 200 verbundenen Filmklemme 400 und einem an der Filmklemme 400 durch eine Steckhalterung 406 angebrachten Indikatorstab 601, auf dem parallel zur Filmebene ein Visierring 604, an dem eine Rechteckblende 612 angebracht werden kann, in der Richtung des langen Schenkels 603 verschiebbar ist.

Für die Anwendung des Filmhalters im Seitenzahnbereich wird das Verbindungsstück 200 durch eine Steckhalterung, die auf der distalen Fläche 101 des Aufbißblocks 100 anfängt und auf der mesialen Fläche 106 endet, am Aufbißblock lösbar fixiert.

Für Meßaufnahmen im Oberkiefer- Seitenzahnbereich wird die obere Steckhalterung verwendet, die auf der distalen Fläche des Aufbißblocks 101 anfängt und auf der mesialen Aufbißblockseite 106 in die obere Öffnung 107 mündet und aus mehreren ineinander übergehenden Perforationen besteht. Für Aufnahmen im Oberkiefer-Seitenzahnbereich können die Filmklemme 400, wie in der Fig. 1 mit der Steckhalterung 405 für das Zahnrad 205 nach unten und mit dem Schlitz 404 nach oben oder die Filmklemme 500 in gleicher Weise verwendet werden.

Für die Anfertigung von Meßaufnahmen im Unterkiefer- Seitenzahnbereich im dritten Quadranten (= Unterkiefer linke Patientenseite) wird der Filmhalter wie in Fig. 1 zusammengesetzt, wobei das Verbindungsstück 200 in die untere Steckhalterung des Aufbißblocks eingesteckt wird, die auf der distalen Fläche 101 anfängt und auf der mesialen Aufbißblockfläche 106 in die untere Öffnung 109 mündet. Die Öffnung 109 besteht aus der gleichen Anzahl von ineinander übergehenden Perforationen wie die obere Öffnung 107. Die Filmklemme 500 (Fig. 10) wird für Aufnahmen im Unterkiefer- Seitenzahnbereich im dritten Quadranten (= Unterkiefer linke Patientenseite) mit der Steckhalterung 505 für das Zahnrad 205 nach oben und mit dem Schlitz 504 nach unten eingesetzt. Die Filmklemme 500 sollte aufgrund ihrer niedrigen Höhe für Meßaufnahmen im Unterkiefer eingesetzt werden, weil der Mundboden der Patienten die Positionierung der höheren Filmklemme 400 wegen Platzmangels nicht erlaubt.

In Fig. 1 sind auf der mesialen Aufbißblockfläche 106 der innere 202 und der äußere Stift 203 des Verbindungsstückes für Seitenzähne 200 zu sehen, weil die Breite des Verbindungsstückes 200 in der Höhe der beiden Stifte 202 und 203 genau soviel beträgt wie die mesio-distale Breite des Aufbißblocks 100. Die Höhe des Aufbißblocks 100 wurde so ausgerechnet, daß beim Zubeißen auf den Aufbißblock die Berührung der Wurzelkanalinstrumentengriffe durch die antagonistischen Zähne nicht erfolgen kann.

Für die Anwendung des Filmhalters im Seitenzahnbereich auf der rechten Patientenseite wird das Verbindungsstück 200 nicht wie in Fig. 1 von der oralen Aufbißblockfläche 112 eingesetzt, sondern von der bukkalen Aufbißblockfläche 111. Der Aufbißblock 100 wird nun von den Seitenzähnen der linken Patientenseite durch Aufbiß fixiert. Den für die rechte Patientenseite zusammengesetzten Filmhalter kann man sich nun spiegelverkehrt zur Fig. 1 vorstellen.

Die Filmklemme 400 wird durch ein Zahnrad 205 in der Steckhalterung 405 fixiert und ermöglicht durch Umstecken in verschiedenen Positionen die Anpassung der Filmebene an unterschiedliche Zahnneigungen. An der Filmklemme sind das Rückschild 401 und die zwei Leisten 402 und 403 angebracht, die zur Aufnahme des Zahnfilmes geeignet sind. Das Rückschild 401 hat eine rechteckige Form und ist flach gestaltet. Die zwei Leisten 402 und 403 sind niedriger gestaltet als das Rückschild 401 und sind ebenfalls abgerundet und flach, um Verletzungen der Mundschleimhaut zu vermeiden.

Um Röntgenaufnahmen in der Paralleltechnik anfertigen zu können, wird die Zentriervorrichtung 600, bestehend aus einem Indikatorstab 601, einem Visierring 604 und einer Rechteckblende 612, in der Steckhalterung 406 der Filmklemme 400 angebracht. An der Filmklemme 400 kann in der Steckhalterung 406 der kurze Schenkel 602 des Indikatorstabes 601 eingesteckt werden, an dem rechtwinklig und starr der lange Schenkel 603 angebracht ist. Entlang des langen Schenkels 603 kann ein Visierring 604 mittels einer rechteckigen Durchbohrung 606, mit denselben Maßen wie der des Indikatorstabes 601, verschieblich angebracht werden. Mit Hilfe des Visierringes 604 wird der Langtubus des Röntgengerätes so ausgerichtet, daß der Zentralstrahl durch die rechteckige Aussparung 615 senkrecht auf die Filmebene bzw. das Rückschild 401 fällt.

Fig. 2 zeigt eine räumliche Ansicht des neuen Filmhalters als Anwendungsbeispiel für die Herstellung von Meßaufnahmen im Oberkiefer- Frontzahnbereich. Das Verbindungsstück 300 wird auf der mesialen Fläche 106 des Aufbißblocks 100 mittels zweier Stifte 302 und 303 (siehe Fig. 6) in die obere Steckhalterung 107 eingesteckt. Die obere Steckhalterung 107 fängt auf der distalen Aufbißblockfläche 101 an und mündet in die obere Öffnung 107 der mesialen Fläche 106. In der Höhe der oberen Öffnung 107 und der unteren Öffnung 109 befinden sich auf der gesamten Länge der mesialen Aufbißblockfläche 106 eine obere runde Einsparung 108 und eine untere runde Einsparung 110. Die zwei runden Einsparungen 108 und 110 haben den gleichen Durchmesser und sollen die Forminkongruenz der runden Verbindungsstücke 200 und 300 und des eckigen Aufbißblocks 100 beseitigen. Am Verbindungsstück 300 wird für Aufnahmen im Oberkiefer- Frontzahnbereich die Filmklemme 400 eingesetzt, weil die oberen Frontzähne lange Wurzeln haben, die die senkrechte Positionierung des Zahnfilmes erfordern und weil meistens der Gaumen die Positionierung einer solch hohen Filmklemme wie die Filmklemme 400 erlaubt.

Für die Anwendung des Filmhalters im Unterkiefer- Frontzahnbereich wird das Verbindungsstück 300 in der untere Öffnung 109 fixiert. Zur Fixierung des Zahnfilmes können beide Filmklemmen 400 oder 500 verwendet werden. Hierbei würde die Steckhalterung der Filmklemmen 405 oder 505 nach oben und die Schlitze 404 und 504 für die Aufnahme des Zahnfilmes nach unten zeigen. Für die Anfertigung von Meßaufnahmen im Frontzahnbereich kann der Filmhalter durch den Aufbiß der Seitenzähne sowohl auf der rechten als auch auf der linken Patientenseite fixiert werden.

Für die senkrechte Positionierung des Zentralstrahls auf die Filmebene bzw. auf das Rückschild 401 oder 501 dient die Zentriervorrichtung 600. Der kurze Schenkel 602 des Indikatorstabes 601 wird ebenso wie in Fig. 1 in die Steckhalterung 406 eingesetzt. Der Visierring 604 wird mittels der Durchbohrung 606 am langen Schenkel 603 des Indikatorstabes 601 angebracht.

Fig. 3 zeigt eine Ansicht von der mesialen Aufbißblockfläche 106. Sie hat eine rechteckige Form, wobei die langen Seiten des Rechtecks waagerecht und die kurzen Seiten senkrecht liegen. Auf dieser Fläche 106 befinden sich zwei zueinander parallele Öffnungen, bestehend aus der gleichen Anzahl von ineinander übergehenden Perforationen. In der Höhe der oberen Öffnung 107 und der unteren Öffnung 109 befinden sich eine obere runde Einsparung 108 und eine untere runde Einsparung 110. Die Einsparungen 108 und 110 verlaufen ebenfalls parallel zueinander. Der Aufbißblock wird aus einem geeignetem Kunststoff oder Werkstoff hergestellt

Fig. 4 zeigt einen Schnitt durch den Aufbißblock 100 in mesio-distaler Richtung. Von der Seite gesehen ist der Aufbißblock 100 trapezförmig, sodaß man insgesamt den Aufbißblock als trapezförmigen Formkörper bezeichnen kann. Von der distalen Aufbißblock- Fläche 101 verlaufen durch eine gemeinsame Öffnung 102 (Fig. 5) zwei Durchbohrungen auf die mesiale Aufbißblockfläche 106. Die obere Durchbohrung 103 verläuft parallel zur oberen Aufbißblockfläche 113 und die untere Durchbohrung 104 verläuft parallel zur unteren Aufbißblockfläche 114. Die Durchbohrungen 103 und 104 verlaufen genauso wie die Oberkiefer- und die Unterkieferzähne bei der Mundöffnungsbewegung -von mesial nach distal betrachtet- keilförmig auseinander und ermöglichen die Anpassung der Filmklemme an die Position der Zähne. In der Höhe der distalen Öffnungen 102 befindet sich eine runde Einsparung 105 mit dem gleichen Durchmesser wie die Einsparungen 108 und 110. Sie erfüllt die gleiche Aufgabe wie die Einsparungen 108 und 110.

Fig. 5 zeigt eine Ansicht auf die distale Aufbißblockfläche 101. In der Mitte der distalen Fläche 101 befindet sich eine Öffnung 102, bestehend aus mehreren ineinander übergehenden Perforationen. Von dieser Öffnung aus verlaufen die zwei in Fig. 4 dargestellten Durchbohrungen 103 und 104 nach distal, die sich zur Aufnahme und Halterung der Stifte 202 und 203 des Verbindungsstückes 200 und der Stifte 302 und 303 des Verbindungsstückes 300 eignen.

Fig. 6 zeigt eine Ansicht von oben auf das Verbindungsstück 200, das für Meßaufnahmen im Seitenzahnbereich verwendet wird. Das Verbindungsstück 200 besteht aus einem Hauptstück 201, an das an einem Ende zwei zueinander parallel verlaufende Stifte angebracht sind: einen inneren 202 und einen äußeren 203 und am anderen Ende das Nebenstück 204. Die Stifte 202 und 203 verlaufen senkrecht zum Hauptstück 201. Der Abstand zwischen den Stiften 202 und 203 ist genauso groß wie der Durchmesser einer einzelnen Perforation aus den Öffnungen 107, 109 oder 102. Dadurch kann die Fixierung des Verbindungsstückes 200 in dem Aufbißblock 100 in mehreren Positionen erfolgen und je nach Kieferbreite eine Längenänderung des Filmhalters bewirken.

Am anderen Ende des Hauptstückes 201 befindet sich das Nebenstück 204, das den gleichen Durchmesser wie das Hauptstück 201 und die beiden Stifte 202 und 203 hat, wobei das Hauptstück 201 und das Nebenstück 204 einen spitzen Winkel zueinander bilden. Das Hauptstück 201, die Stifte 202 und 203 und das Nebenstück 204 liegen in der gleichen Ebene. Senkrecht zu der Längsachse des Nebenstückes 204 ist das Zahnrad 205 angebracht, das einen größeren Durchmesser als das Nebenstück 204 hat. Das Zahnrad 205 ermöglicht die Fixierung des Verbindungsstückes 200 in den Steckhalterungen 405 und 505 der beiden Filmklemmen 400 und 500 in verschiedenen Positionen, sodaß der Zahnfilm parallel zur Zahnlängsachse positioniert werden kann.

Fig. 7 zeigt eine Ansicht von oben auf das Verbindungsstück 300, das für Meßaufnahmen im Frontzahnbereich verwendet wird. Das Verbindungsstück 300 besteht aus einem Hauptstück 301, das an einem Ende zwei zueinander parallel verlaufende Stifte hat: einen inneren 302 und einen äußeren 303 und am anderen Ende das Zahnrad 304. Beide Stifte 302 und 303 verlaufen senkrecht zum Hauptstück 301 und haben den gleichen Durchmesser wie das Hauptstück 301, das Hauptstück 201, das Nebenstück 204 und die Stifte 202 und 203. Das Hauptstück 301 und die Stifte 302 und 303 liegen in der gleichen Ebene. Senkrecht auf die Längsachse des Hauptstückes 301 ist das Zahnrad 304 angebracht. Das Zahnrad 304 hat die gleiche Anzahl von Zähnen und den gleichen Durchmesser wie das Zahnrad 205. Es dient ebenfalls zur Fixierung der Filmklemmen 400 und 500 mittels der Steckhalterungen 405 und 505 an das Verbindungsstück 300.

Fig. 8 zeigt einen Querschnitt der Zahnräder 205 und 304 im Vergleich zum Durchmesser der Hauptstücke 201 und 301. Der Durchmesser der beiden Zahnräder 205 und 304 ist größer als der Durchmesser der Hauptstücke 201 und 301 und hat mehrere Zähne, die zum einen die rotationssichere Fixierung der Verbindungsstücke 200 und 300 an die Filmklemmen 400 und 500 und zum anderen durch Umstecken die Anpassung der Filmebene an die Zahnlängsachsen ermöglichen.

Fig. 9 zeigt einen Schnitt durch die Mitte der Filmklemme 500. In dieser Schnittebene sind nur das Rückschild 501, die vordere Fläche des Rückschildes 501, die zusammen mit den seitlichen Leisten 502 und 503 den Schlitz 504 zur Aufnahme des Zahnfilmes bilden, die rechteckige Steckhalterung 506 zur Aufnahme des kurzen Schenkels 602 des Indikatorstabes 601, die Steckhalterung 505 zur Aufnahme der Zahnräder 205 oder 304, die rillenförmige Filmauflagefläche 507, in der sich die untere Kante des Zahnfilmes befindet und die vor der rillenfömigen Filmauflagefläche 507 sich befindliche waagerechte Leiste 510. In dieser Zeichnung ist gestrichelt dargestellt die Position einer der Leisten (502 oder 503) im Verhältnis zu den anderen Bestandteilen der Filmklemme 500. Dadurch wird die Lage des Schlitzes 504 zwischen dem Rückschild 501 und den beiden Leisten 502 und 503 eindeutig. Ebenfalls wird die genaue Position der rechteckigen Steckhalterung 506 eindeutig: diese Steckhalterung liegt unterhalb des Schlitzes 504 und nimmt auch den vorderen Anteil der Unterseite des Rückschildes 501 ein. Die Steckhalterung 506 für die Aufnahme des Zahnrades 205 oder 304 liegt auf der Rückseite des Rückschildes 501 und der Steckhalterung 505 und ihr inneres Profil, in das die Zahnräder 205 oder 304 passen, ist so angeordnet, daß in der Höhe der rillenförmigen Filmauflagefläche 507 die waagerechte Achse des Zahnrads durch die Spitze eines einzelnen Zahnes verläuft. Der Sinn dieser Anordnung ist, daß die Filmklemme 500 durch die Steckhalterung 505 am Zahnrad 205 oder 304 so fixiert ist, daß die senkrechte Lage des Filmes zur Horizontalen genau definiert ist. Anhand dieser senkrechten Filmklemmen-position kann die Achsenneigung der einzelnen Zähne genauer bestimmt werden und die Filmklemmenachse dementsprechend eingestellt werden

Fig. 10 zeigt eine Frontalansicht der Filmklemme 500, die sich für die waagerechte Positionierung des Zahnfilmes eignet. Das Rückschild 501 hat eine rechteckige Form, wobei die längere Seite 508 waagerecht und die kürzere Seite 509 senkrecht liegt. Das Rückschild 501 ist in seinen Ausmaßen kleiner gestaltet als ein Zahnfilm, so daß die Ränder handelsüblicher Zahnfilme für Erwachsene über die Ränder des Rückschildes 501 hinausragen und eine kontrollierbare Verbiegung des Zahnfilmes beim Berühren von Strukturen der Mundhöhle stattfinden kann. Die obere linke und rechte Ecke der Filmklemme 500 sind genauso wie alle ihre Kanten abgerundet, ermöglichen somit die Anpassung der Filmklemme an die runden Strukturen der Mundhöhle und verhindern Verletzungen der Schleimhaut.

Vor dem Rückschild 501 befinden sich seitlich zwei Leisten: eine rechte 502 und eine linke 503, die eine Verbiegung des Zahnfilmes zum Zahn hin vermeiden und den Zahnfilm intraoral im Schlitz 504 stabilisieren. Die Leisten 502 und 503 schließen seitlich mit dem Rückschild 501 ab und sind so schmal und flach zu gestalten, daß das Material aus dem sie geformt sind, eine ausreichende Elastizität zuläßt, ohne daß es abbricht. Es soll vorzugsweise Kunststoff für die Herstellung der Filmklemme 500 verwendet werden. Die Leisten 502 und 503 sind niedriger gestaltet als die kurzen senkrechten Seiten des Rückschilds 501, erlauben daher die Anpassung der Filmklemme an die runden Strukturen der Mundhöhle, wie z.B. an den Gaumen und ermöglichen die Einhaltung eines geringen Film-Objekt-Abstands. Die oberen Ecken dieser beiden Leisten 502 und 503 und ihre Kanten sind aus dem gleichen Grund wie der Rückschild 501 abgerundet. Die Leisten 502 und 503 sind durch eine waagerechte Leiste 510 miteinander verbunden, die höher als die Filmauflagerille 507 -hier gestrichelt dargestellt- ist, sodaß die Filmauflagerille 507 von vorne nicht zu sehen ist. Die Übergänge der waagerechten Leiste 510 zu den seitlichen Leisten 502 und 503 sind abgerundet.

Unterhalb des Rückschildes 501 und der Leisten 502 und 503 verläuft -gestrichelt dargestellt- die im Durchmesser rechtwinklige Steckhalterung 506 auf der gesamten Breite der Filmklemme 500 und parallel zur Unterseite der Filmklemme 500. Sie eignet sich zur Aufnahme des kurzen Schenkels 602 des Indikatorstabes 601.

Auf der Rückseite des Rückschildes 501 und der Steckhalterung 506 und in der Mitte dieser beiden Strukturen befindet sich -ebenfalls gestrichelt dargestellt- die Steckhalterung 505 für die Aufnahme der Zahnräder 205 oder 304. Die Steckhalterung 505 hat dieselbe Breite wie die beiden Zahnräder 205 und 304 und ist senkrecht zur Rückseite des Rückschilds 501 angebracht, d. h. wenn die Zahnräder 205 oder 304 in der Steckhalterung 505 eingesetzt sind, verläuft die Rotationsachse/Längsachse des Zahnrads parallel zum Rückschild 501.

Fig. 11 zeigt eine Ansicht von vorne auf die Filmklemme 400, die sich zur senkrechten/Hochkant-Positionierung des Zahnfilmes eignet. Die Filmklemme 400 ist schmaler und höher gestaltet als die Filmklemme 500 und hat auch eine rechteckige Form, wobei die kurze Seite 408 des Rückschildes 401 waagerecht und die lange Seite 409 senkrecht liegt. Die Maße des Rückschildes 401 sind kleiner als ein handelsüblicher Zahnfilm für Erwachsene und ermöglichen ebenfalls die kontrollierbare Verbiegung des Filmes beim Berühren von intraoralen Strukturen. Vor dem Rückschild 401 befinden sich seitlich zwei Leisten 402 und 403, die niedriger als das Rückschild 401 gestaltet sind und seitlich mit dem Rückschild 401 abschließen. Das Rückschild 401 und die Leisten 402 und 403 haben abgerundete Kanten und obere Ecken und sind ebenfalls so schmal und flach zu gestalten, daß das Material aus dem sie hergestellt sind eine ausreichende Elastizität zuläßt ohne daß es abbricht. Auch für die Herstellung dieser Filmklemme 400 soll vorzugsweise Kunststoff verwendet werden. Die oben beschriebene Gestaltung der beiden Leisten 402 und 403 und des Rückschildes 401 soll zur Anpassung des Filmhalters an die runden Strukturen der Mundhöhle und zur Vermeidung von Schleimhautverletzungen oder Würgereizentstehung beitragen. Die seitlichen Leisten 402 und 403 sind durch die waagerechte Leiste 410 miteinander verbunden, die höher gestaltet ist als die rllenförmigen Filmauflagefläche 407 und somit von vorne nicht zu sehen ist. Die Übergänge zwischen den seitlichen Leisten 402 und 403 und der waagerechten Leiste 410 sind ebenfalls abgerundet.

Unter dem Rückschild 401 und den beiden Leisten 402 und 403 befindet sich die im Durchmesser ebenfalls rechtwinklige Steckhalterung 406 für die Aufnahme des kurzen Schenkels 602 des Indikatorstabes 601, die sich auf die gesamte Breite der Filmklemme 401 erstreckt und parallel zur Unterseite der Filmklemme 400 verläuft. Sie ist gestrichelt dargestellt.

Auf der Rückseite des Rückschildes 401 und der Steckhalterung 406 und in der Mitte dieser beiden Strukturen befindet sich -ebenfalls gestrichelt dargestellt- die Steckhalterung 405 für die Aufnahme der Zahnräder 205 und 304, die einen Durchmesser passend zum Durchmesser der beide Zahnräder hat. Diese Steckhalterung 405 ist ebenfalls senkrecht auf der Rückseite des Rückschildes 401 angebracht.

Fig. 12 zeigt eine Ansicht auf den Visierring 604 von der tubusnahen Seite. Der Visierring 604 wird am langen Schenkel 603 des Indikatorstabes 601 mittels einer Befestigungsvorrichtung 605 angebracht, in der sich eine rechteckige Durchbohrung 606 passend zum Querschnitt des langen Schenkels 603 befindet. Die Befestigungsvorrichtung 605 ist aus dem gleichen Material gebaut wie der äußere tubusnahe Ring 607 und der innnere tubusferne Ring 611. Der äußere tubusnahe Ring 607 des Visierringes 604 hat einen größeren Querschnitt wie der innere tubusferne Ring 611 und hat auf der tubusnahen Seite eine runde Markierung 608 für die Positionierung eines runden Langtubus und eine eckige Markierung 609 für die Positionierung eines rechteckigen Langtubus. An der unteren Innenseite des tubusnahen Ringes befindet sich eine kleine erhobene Markierung 610, die zur Fixierung der Rechteckblende 612 entweder in waagerechter oder senkrechter Lage dient. Der äußere tubusnahe Ring ist unbeweglich mit dem inneren tubusfernen Ring 611 fixiert. Der innerer tubusferne Ring 611 hat einen kleineren Durchmesser als der äußere tubusnahe Ring 607 und dient zur Fixierung der Rechteckblende 612 im Visierring 604.

Die Aufgabe der Zentriervorrichtung ist, die Röntgenstrahlung auf den zu untersuchenden Zahn zu positionieren, sodaß keine Fehlprojektionen und somit eine erhöhte Strahlenbelastung für den Patienten erfolgt. Die Strahlenbelastung für den Patienten kann jedoch weiterhin durch einen weiteren Bestandteil des Filmhaltersatzes, der in Fig. 13 dargestellt ist, gesenkt werden

Fig. 13 zeigt eine Ansicht auf die Rechteckblende 612 von der tubusnahen Seite. Die Rechteckblende 612 hat den gleichen Durchmesser wie der innere Durchmesser des äußeren tubusnahen Ringes 607, sodaß die Rechteckblende 612 in den Ring 607 eingesteckt werden kann. In der Mitte der Rechteckblende 612 befindet sich eine rechteckige Perforation 615 von der Größe eines handelsüblichen Zahnfilmes für Erwachsene, die deckungsgleich mit dem in der Filmklemme 400 oder 500 eingesteckten Zahnfilm ist. Da die Rechteckblende 612 aus einem für Röntgenstrahlen undurchsichtigen Material hergestellt werden soll, ist die rechteckige Aussparung 615 die einzige Stelle der Rechteckblende, die Röntgenstrahlen durchläßt. Dadurch wird nur der Zahnfilm belichtet. Die Folge ist eine maximale Reduktion der Strahlenbelastung für den Patienten.

Am unteren Rand der Rechteckblende 612 unterhalb der waagerechten Seite 616 der rechteckigen Aussparung 615 und in der Mitte derselben befindet sich eine zur Markierung 610 passende Aussparung 614 für die senkrechte Positionierung der Rechteckblende, um Meßaufnahmen in Verbindung mit der Filmklemme 400 anzufertigen. Ebenfalls am Rand der Rechteckblende in der Höhe der senkrechten Seite 617 der rechteckigen Aussparung 615 und in der Mitte derselben befindet sich eine zweite zur Markierung 610 passende Aussparung 613 für die waagerechte Positionierung der Rechteckblende 612, um Meßaufnahmen in Verbindung mit der Filmklemme 500 anzufertigen.

Fig. 14 zeigt einen Schnitt durch die Mitte des Visierringes 604 bei eingesetzter Rechteckblende 612. Darauf sind die unterschiedlichen Durchmesser des inneren tubusfernen Ringes 611 und des äußeren tubusnahen Ringes 607 dargestellt. Der innere tubusferne Ring 611 hat einen kleineren Durchmesser als der äußere tubusnahe Ring 607 und erfüllt somit die Aufgabe der Retention für die Rechteckblende 612. So kann die Rechteckblende 612 nur von der Seite des äußeren tubusnahen Ringes 607 eingesteckt werden.

An der Innenseite des äußeren tubusnahen Ringes 607 ist die untere erhobene bis zum Rand des inneren tubusfernen Ringes 611 reichende Markierung 610 - gestrichelt dargestellt- sichtbar. In der Mitte der Rechteckblende 612 ist ebenfalls - gestrichelt dargestellt- die rechtwinklige Aussparung 615 sichtbar.

Fig. 15 zeigt eine schaubildliche Ansicht des neuen Zahnfilmhalters als Anwendungsbeispiel für die Durchführung von Meßaufnahmen in der Paralleltechnik im dritten Quadranten. Die gestrichelt gezeichnete Filmklemme 1300 zeigt die Positionierung der selben bei Meßaufnahmen im zweiten Quadranten. Damit der Zentralstrahl senkrecht und mittig auf den Zahnfilm fällt, muß auch die Zentriervorrichtung für den Röntgentubus nach oben in Richtung des Pfeils umgestellt werden. Der dargestellte Zahnfilmhalter besteht aus einem Aufbißblock 1100, einem kleinen - auf einer Seite mit dem Aufbißblock 1100 und auf der anderen Seite mit der Filmklemme 1300 bzw. 1400 verbunden - Verbindungsstück 1200, einer in der Längsrichtung des Aufbißblocks 1100 verlaufenden Führungsschiene 1500 für die Fixierung der Zentriervorrichtung 1600, einer Zentriervorrichtung 1600 für den Röntgentubus und einer in der Zentriervorrichtung 1600 anzubringenden Rechteckblende 1700.
Der Aufbißblock 1100 ist rechteckig. Seine obere Fläche 1101 und die untere Fläche 1102, die mesiale Fläche 1103 und die distale Fläche 1104, die vordere Fläche 1105 und die hintere Fläche 1106 sind jeweils parallel zueinander. Dabei ist die Breite 1107 des Aufbißblocks 1100 kleiner als die Höhe 1108 des Aufbißblocks 1100, die wiederum kleiner als die Länge 1109 des Aufbißblocks 1100 ist. Die Strecke 1109 beträgt mehr als eine vestibulo-oralen Molarenbreite, damit die Fixierung des Aufbißblocks 1100 per Aufbiß optimiert wird. Die Höhe 1108 des Aufbißblocks 1100 ist so bemessen, daß beim Aufbeißen auf den Aufbißblock 1100 während der Meßaufnahme ein Kontakt der im Zahn fixierten Wurzelkanalinstrumente zu den Zähnen im Gegenkiefer verhindert wird.
Der Aufbißblock ist nach vestibulär mit der Führungsschiene 1500 und nach oral mit dem kleinen Verbindungsstück 1200 unlösbar verbunden.
Das kleine Verbindungsstück 1200 ist annähernd trapezförmig gestaltet und ist mit seiner breiten Fläche 1203 senkrecht auf die mesiale Fläche 1103 des Aufbißblocks 1100 angebracht. Seine vordere Fläche 1201 und die hintere Fläche 1202 haben die gleichen Maße und verlaufen parallel zueinander. Die Fläche 1203 des Verbindungsstückes 1200 ist unlösbar mit dem Aufbißblock 1100 fixiert und ist größer als die Fläche 1204. Die obere 1205 und die untere Fläche 1206 des Verbindungsstückes 1200 sind in ihren Maßen gleich, aber sie verlaufen nicht parallel zueinander, sondern aufeinander zu, treffen sich aber nicht. Am spitzen Ende des Verbindungsstückes 1200 und vor der Strecke 1204 befindet sich die Öffnung 1205 für die Steckhalterung 1305 bzw. 1405 der Filmklemmen 1300 und 1400. Die Öffnung 1205 durchquert beide Flächen 1201 und 1202 und verläuft mit ihrer Längsachse parallel zu den Fläche 1103 und 1104 des Aufbißblocks 1100. Die Filmklemmen 1300 und 1400 sind dank dieser Verbindung lösbar am kleinen Verbindungsstück 1200 angebracht. Sie lassen sich in mehreren Positionen einsetzen, um erstens Aufnahmen sowohl im Seiten-, als auch im Frontzahnbereich durch den Einsatz beider zu ermöglichen, zweitens wird durch das Einrasten der jeweiligen Filmklemme in der gewünschten Position die intraorale Lage der selben während der Meßaufnahme gesichert. Beide Steckhalterungen 1305 und 1405 befinden sich jeweils in einem Verlängerungsstück 1304 bzw. 1404 der Filmklemmen. Die Filmklemme 1300 für die Seitenzähne ist rechteckig, kleiner als ein handelsüblicher Zahnfilm und hat abgerundete Ecken, um einer Verletzung der intraoralen Weichteile zu vermeiden. Der Zahnfilm wird zwischen dem Rückschild 1301 und dem Frontschild 1302 fixiert. Beide Schilde 1301 und 1302 haben auf der Seite des Verlängerungsstückes 1305 eine Form, die sich der Form der Flächen 1205 bzw. 1206 anpaßt. Die dem Verlängerungsstück 1304 entgegengesetzte Kante 1307 hat einen geradlinigen Verlauf. Zwischen den beiden Schildern 1301 und 1302 befindet sich eine rillenförmige Filmauflagefläche 1303, in der der Zahnfilm beim Fixieren in der Filmklemme aufliegen sollte.
In der Verlängerung der Längsachsen des Aufbißblocks 1100 und parallel zu den Flächen 1103, 1104, 1105 und 1106 verläuft die Führungsschiene 1500, die zur Fixierung der Zentriervorrichtung 1600 für den Röntgentubus dient. Die Führungsschiene 1500 ist unlösbar mit dem Aufbißblock verbunden und ist mittig auf der Fläche 1105 angebracht. Auf ihrer Außenseite in der Längsrichtung befindet sich eine erhobene Leiste 1501, in die als Gegenstück die Rillen 1601a und 1601b des Indikatorstabes 1600 greifen. Aus dieser Weise wird die Zentriervorrichtung 1600 je nach Positionierung der Filmklemme für den Ober- oder Unterkiefermeßaufnahmen eingestellt.
Die Zentriervorrichtung 1600 besteht aus einem Stab 1601, einem Visierring 1603 und einer Verbindung 1602 zwischen den ersten beiden.
Der Stab 1601 ist innen hohl und seine Innenseite entspricht der Gegenform der Führungsschiene 1500. Am Stab 1601 ist durch eine unlösbare Verbindung 1602 ein Visierring angebracht, der kreisförmig ist und den gleichen Durchmesser hat wie ein handelsübliches Röntgenlangtubus. Der Visierring 1603 ist senkrecht auf der Führungsschiene 1500 und den Stab 1601 und parallel zu den Flächen 1201, 1202 und zu den Flächen 1301, 1302, 1401 und 1402 der Filmklemmen 1300 und 1400 angebracht. Auf der Innenseite des Visierringes 1603 befindet sich die erhobene Markierung 1604, mit deren Hilfe die Rechteckblende 1700 in mehreren Positionen fixierbar ist. Der Visierring 1603 soll immer deckungsgleich zu der eingestellten Filmklemme sein, so daß bei korrekter senkrechter Einstellung der Röntgen-Röhre auf den Zahnfilm, der Zentralstrahl mittig auf diesen trifft und somit eine geringe und gleichmäßige Vergrößerung des Zahnes stattfindet. Dadurch wird der Zahn auf dem Zahnfilm sehr scharf abgebildet und führt schließlich zur Bestimmung einer korrekten Arbeitslänge - als erklärtes Ziel der Röntgen-Meßaufnahme.
Die Rechteckblende 1700 hat genau wie der Visierring 1603 einen runden Querschnitt und in der Mitte eine rechteckige Aussparung von der Größe eines handelsüblichen Zahnfilmes. Die Rechteckblende 1700 wird in den Visierring 1603 eingesteckt und hat somit eine Klemmpassung. Die erhobene Markierung 1604 und die zu ihr passenden Aussparungen 1702 und 1703 sollen eine bestimmte, für den zu untersuchenden Zahn ausgewählte Rechteckblende-Position während der Meßaufnahme sichern.

Fig. 16 zeigt eine frontale Ansicht auf die Filmklemme 1300 für die waagerechte Positionierung des Zahnfilmes. Das Rückschild 1301 hat eine rechteckige Form, wobei die längere Seite waagerecht und die kürzere Seite senkrecht liegt. Das Rückschild 1301 ist in seinen Ausmaßen kleiner gestaltet als ein Zahnfilm, so daß die Ränder handelsüblicher Zahnfilme für Erwachsene über die Ränder des Rückschildes hinausragen und eine kontrollierbare Verbiegung des Zahnfilmes beim Berühren von Strukturen der Mundhöhle stattfinden kann. Zusätzlich sind die Ränder der Filmklemme 1300 abgerundet, um einer Verletzung der intraoralen Weichteile entgegenzuwirken. Das Frontschild 1302 ist etwas kleiner als das Rückschild 1301. Zwischen diesen beiden Schildern wird der Zahnfilm während der Meßaufnahme fixiert, wobei die Zahnfilmkante in der Filmauflagerille 1303 aufliegen soll. Oberhalb der Filmauflagerille 1303 und in der Mitte der langen Kante 1306 der Filmklemme 1300 befindet sich die Halterung 1304 mit der Steckhalterung 1305 für die Öffnung 1204 des kleinen Verbindungsstückes 1200. Die Halterung 1304 hat eine rechteckige Form und seine Höhe 1308 ist nicht länger als die Fläche 1204. Alle Bestandteile der Filmklemme 1300 sind möglichst schmal und flach zu gestalten, damit sie intraoral gut zu positionieren sind, ohne ein Würgereiz zu verursachen. Die Kante 1307 der Zahnfilmklemme 1300 hat einen geradlinigen Verlauf, während die Kante 1306 sich der Form der Flächen 1205 und 1206 anpaßt.

Fig. 17 zeigt eine frontale Ansicht auf die Filmklemme 1400 für die waagerechte Positionierung des Zahnfilmes. Das Rückschild 1401 hat eine rechteckige Form, wobei die kürzere Seite waagerecht und die längere Seite senkrecht liegt. Das Rückschild ist in seinen Ausmaßen kleiner gestaltet als ein Zahnfilm, so daß die Ränder handelsüblicher Zahnfilme für Erwachsene auch hier über die Ränder des Rückschildes hinausragen und eine kontrollierbare Verbiegung des Zahnfilmes beim Berühren von Strukturen der Mundhöhle stattfinden kann. Die Kanten der Filmklemme 1400 sind ebenfalls abgerundet. Das Frontschild 1402 ist etwas kleiner als das Rückschild 1401. Zwischen diesen beiden Schildern wird der Zahnfilm während der Meßaufnahme fixiert, wobei die der Steckhalterung 1405 entgegengesetzte Zahnfilmkante in der Filmauflagerille 1403 liegen soll. Oberhalb der Filmauflagerille 1403 und in der Mitte der schmalen Seite 1406 der Filmklemme 1400 befindet sich auch hier die Halterung 1404 mit der Steckhalterung 1405 für die Öffnung 1204 des kleinen Verbindungsstückes 1200. Die Kante 1408 überschreitet nicht die Länge der Kante 1204 des Verbindungsstückes 1200. Alle Bestandteile der Filmklemme 1400 sind möglichst schmal und flach zu gestalten, damit sie intraoral gut zu positionieren sind, ohne ein Würgereiz zu verursachen. Die Kante 1407 der Zahnfilmklemme 1300 hat einen geradlinigen Verlauf, während die Kante 1406 sich der Form der Flächen 1205 und 1206 anpaßt.

Fig. 18 zeigt eine Ansicht der Steckhalterung 1305 der Filmklemme 1300, die in die Öffnung 1205 reingreift. Das Einsetzen der Steckhalterung 1305 in die Öffnung 1204 ist nur von der Öffnung 1205b möglich. Die Steckhalterung 1305 ist annähernd zylinderförmig, hat aber in der Mitte eine Einziehung 1305b, die verhindert, daß die in der Öffnung 1205 bereits eingesetzte Steckhalterung 1305 unabsichtlich rausgleitet. Die Spitze der Steckhalterung 1305 ist in der Mitte durch eine Nut 1305a geteilt, die eine gewisse Elastizität der beiden Spitzenhälften 1305c und 1305d gewährleistet. Dadurch läßt sich die Steckhalterung 1305 in die Öffnung einstecken und kann durch diese arretierbaren Spitzenhälften die Einengung 1205d der Öffnung 1205 passieren. Auf der Innenseite der Öffnung 1205 befinden sich zwei erhobene Markierungen 1205c und 1205d, die das Einrasten der Nut 1305a in zwei unterschiedliche Positionen sichert. Das Gleiten der Steckhalterung 1305 von einer Position in die andere ist durch die Rotation möglich. Die Rotationsebene verläuft parallel zu den Flächen 1201 und 1202.
Die Steckhalterung 1405 der Zahnfilmklemme 1400 besteht aus den gleichen Bestandteilen.

Fig. 19 zeigt eine Ansicht auf den Visierring 1603 von der tubusnahen Seite. Der Visierring 1603 wird mit Hilfe einer Verbindung 1602 am Tubus 1601 unlösbar fixiert. Das Verbindungsstück 1602 hat einen runden inneren Querschnitt, der vom Durchmesser her größer als der Querschnitt der Führungsstange 1500 ist, die in diesen Tubus reingreift. An der Außenseite der Führungsschiene 1500 befindet sich eine erhobene Markierung 1501, die bei Meßaufnahmen im dritten und im ersten Quadranten entsprechen der Fig. 15 in die Rille 1601b reingreift Bei Meßaufnahmen im vierten und im zweiten Quadranten greift die erhobene Markierung in die Rille 1601a. Dadurch wird die Position des Visierringes 1603 von unten für Aufnamen im Unterkiefer, nach oben für Aufnahmen im Oberkiefer variiert. Der äußere tubusnahe Ring 1607 des Visierringes 1603 hat einen größeren Querschnitt als der innere tubusferne Ring 1608 und bietet somit einen Stop für die Rechteckblende 1700. Der Visierring hat auf der tubusnahen Seite eine runde Markierung 1606 für die Positionierung eines Langtubus und eine eckige Markierung 1605 für die Positionierung eines eckigen Langtubus. An der inneren Seite des tubusnahen Ringes befindet sich eine kleine erhobene Markierung 1604, die zur Fixierung der Rechteckblende entweder in waagerechter oder senkrechter Position dient. Der äußere tubusnahe Ring 1607 ist unbeweglich mit dem inneren tubusfernen Ring 1608 fixiert. Die Rechteckblende 1700 hat den gleichen Durchmesser wie der innere Durchmesser des äußeren tubusnahen Ringes 1607, so daß die Rechteckblende 1700 in den Ring 1607 durch leichte Klemmpassung fixiert ist.

Fig. 20 zeigt eine schaubildliche Ansicht auf die Rechteckblende 1700. In der Mitte dieser Rechteckblende befindet sich eine rechteckige Aussparung von der Größe eines handelsüblichen Zahnfilmes für Erwachsene, die bei korrekter Positionierung des Visierringes zur Filmklemme deckungsgleich mit dem in der Filmklemme 1300 und 1400 einsteckten Zahnfilm ist. Da die Rechteckblende aus einem röntgenundurchlässigen Material hergestellt wird, ist die Aussparung 1701 die einzige Stelle der Rechteckblende, die Röntgenstrahlung durchläßt. Dadurch wird nur der Zahnfilm belichtet. Die Folge ist eine maximale Reduktion der Strahlenbelastung für den Patienten. Am unteren Rand der Rechteckblende 1701 unterhalb der schmalen Seite 1702 der Aussparung 1701 und in der Mitte derselben befindet sich eine zur Markierung 1604 des Visierringes 1603 passende Aussparung 1705 für die senkrechte Positionierung der Rechteckblende, um Meßaufnahmen in Verbindung mit der Filmklemme 1400 anzufertigen. Ebenfalls am Rand der Rechteckblende in der Höhe der langen Seite 1703 und in der Mitte derselben befindet sich eine zweite zur Markierung 1604 passenden Aussparung 1704 für die waagerechte Positionierung der Rechteckblende 1701, um Meßaufnahmen mit der Filmklemme 1300 anzufertigen.

Fig. 21 zeigt einen Schnitt durch die Mitte des Visierringes 1603 bei eingesetzter Rechteckblende 1700. Darauf sind die unterschiedlichen Durchmesser des inneren tubusfernen Ringes 1608 und des äußeren tubusnahen Ringes 1607 dargestellt. Der innere tubusfene Ring 1608 hat einen kleineren Durchmesser als der äußere tubusnahe Ring 1607 und erfüllt die Aufgabe der Retention für die Rechteckblende 1700. So kann die Rechteckblende nur von der Seite des äußeren tubusnahen Ringes 1607 eingesteckt werden. Die erhobene Markierung 1604 - gestrichelt dargestellt - am inneren Ring 1604 dient nicht zur Retention, sondern bestimmt lediglich die Ausrichtung der rechteckigen Aussparung 1701 mit der längeren Kante in vertikaler oder in horizontaler Richtung. In der Mitte der Rechteckblende ist ebenfalls gestrichelt dargestellt die rechteckige Aussparung 1701 sichtbar.

### Filmhalter für die Anfertigung von Meßaufnahmen bei angelegtem Kofferdamrahmen

### Auflistung aller Komponenten des neuen Filmhalters

- **100:**: **Aufbißblock,**
- 101:: distale (dorsale) Aufbißblockfläche,
- 102: distale Öffnung für mehrere ineinander übergehende Durchbohrungen,
- 103:: obere, parallel zur oberen Fläche verlaufende Durchbohrungen für die Fixierung des Verbindungsstückes bei Oberkiefer-Aufnahmen,
- 104:: untere, parallel zur unteren Aufbißblockfläche verlaufende Durchbohrungen für die Fixierung des Verbindungsstückes bei Unterkiefer-Aufnahmen,
- 105:: runde, distal gelegene Einsparung entlang von 102 für die bessere Anpassung des Verbindungsstückes 200 am Aufbißblock 100,
- 106:: mesiale (vordere) Aufbißblockfläche,
- 107:: runde, mesial gelegene, obere Öffnung von 103,
- 108:: obere, runde Einsparung entlang von 107,
- 109:: runde, mesial gelegene, untere Öffnung von 104,
- 110:: untere, runde Einsparung entlang von 109,
- 111:: glatte bukkale Aufbißblockfläche,
- 112:: glatte orale Aufbißblock-Fläche,
- 113:: obere, gerillte Aufbißblockfläche,
- 114:: untere, gerillte Aufbißblockfläche,

- **200:**: **Verbindungsstück für die Seitenzähne**
- 201:: Hauptstück,
- 202:: innerer, senkrecht zum 201 verlaufender Stift,
- 203:: äußerer, senkrecht zum 201 und parallel zum 202 verlaufender Stift,
- 204:: Nebenstück, bildet mit 201 einen spitzen Winkel,
- 205:: Zahnrad zur Anpassung der Filmklemme an die Zahnlängsachsen.

- **300:**: **Verbindungsstück für die Frontzähne,**
- 301:: Hauptstück,
- 302:: innerer, senkrecht zum 301 verlaufender Stift,
- 303:: äußerer, senkrecht zum 301 und parallel zum 302 verlaufender Stift,
- 304:: Zahnrad zur Anpassung der Filmklemme an die Zahnlängsachsen, vom gleichen Durchmesser wie 205.

- **400:**: **Filmklemme für die senkrechte Positionierung des Zahnfilmes,**
- 401:: Rückschild der Filmklemme,
- 402:: rechter, vorderer Stift zur Verhinderung der Abklappung des Filmes nach vorne,
- 403:: linker, vorderer Stift zur Verhinderung der Abklappung des Filmes nach vorne,
- 404:: Schlitz zwischen dem Rückschild und den beiden vorderen Stifte zur Aufnahme des Zahnfilmes,
- 405:: Steckhalterung für die Aufnahme des Zahnrads,
- 406:: rechteckige Steckhalterung für die Aufnahme des Indikatorstabes.
- 407:: Filmauflagerille,
- 408:: waagerechte Fläche des Rückschildes 401,
- 409:: senkrechte Fläche des Rückschildes 401,
- 410:: waagerechte Verbindungsleiste zwischen 402 und 403.

- **500:**: **Filmklemme für die waagerechte Positionierung des Filmes,**
- 501:: Rückschild der Filmklemme,
- 502:: rechter, vorderer Stift zur Verhinderung der Abklappung des Filmes nach vorne,
- 503:: linker, vorderer Stift zur Verhinderung der Abklappung des Filmes nach vorne,
- 504:: Schlitz zwischen dem Rückschild und den beiden vorderen Stifte zur Aufnahme des Zahnfilmes,
- 505:: Steckhalterung für die Aufnahme des Zahnrades,
- 506:: rechteckige Steckhalterung für die Aufnahme des kurzen Schenkels 602 des Indikatorstabes 601,
- 507:: Filmauflagerille,
- 508:: waagerechte Fläche des Rückschildes 501,
- 509:: senkrechte Fläche des Rückschildes 501,
- 510:: waagerechte Verbindungsleiste zwischen 502 und 503.

- **600:**: **Zentriervorrichtung,**
- **601:**: **Indikatorstab,**
- 602:: kurzer Schenkel des Indikatorstabes zur Befestigung an den Filmklemmen 400 oder 500,
- 603:: langer Schenkel des Indikatorstabes zur Aufnahme des Visierringes 604,
- **604:**: **Visierring,**
- 605:: Befestigungsvorrichtung am Visierring für die Positionierung der Röntgenröhre,
- 606:: rechteckige Steckhalterung für den langen Schenkel 602 des Indikatorstabes 601,
- 607:: äußerer, tubusnaher Ring des Visierringes mit
- 608:: Markierung für die Positionierung eines runden Langtubus,
- 609:: eckige Markierung am Visierring für die Positionierung eines rechteckigen Langtubus,
- 610:: untere, mittige Markierung für die Fixation der Rechteckblende,
- 611:: innerer, tubusferner Ring des Visierringes,
- **612:**: **Rechteckblende** mit
- 613:: Markierung für die waagerechte Positionierung der Rechteckblende.
- 614:: Markierung für die senkrechte Positionierung der Rechteckblende,
- 615:: rechteckige Aussparung in der Rechteckblende von der Größe eines handelsüblichen Zahnfilmes für Erwachsene,
- 616:: waagerechter Schenkel der rechtwinkligen Aussparung 615
- 617:: senkrechter Schenkel der rechteckigen Aussparung 615

### Auflistung aller Komponenten der Ausführungsform

- **1100:**: **Aufbißblock mit**
- 1101:: obere Aufbißblockfläche,
- 1102:: untere Aufbißblockfläche,
- 1103:: seitliche Aufbißblockfläche, zeigt je nach Positionierung des Filmhalters entwe der nach mesial oder nach distal. In Fig. 15 liegt diese Fläche mesial zum untersuchten Zahn,
- 1104:: seitliche linke Aufbißblockfläche, zeigt je nach Positionierung des Filmhalters entweder nach mesial oder nach distal,
- 1105:: vordere Aufbißblockfläche,
- 1106:: hintere Aufbißblockfläche,
- 1107:: Breite des Aufbißblocks,
- 1108:: Höhe des Aufbißblocks,
- 1109:: Länge des Aufbißblocks.

- **1200:**: **Verbindungsstück zwischen dem Aufbißblock 1100 und den Filmklemmen**
- **1300**: **bzw. 1400 mit**
- 1201:: vordere, innere Fläche,
- 1202:: hintere, äußere Fläche,
- 1203:: Kontaktfläche des Verbindungsstückes 1200 zu der Aufbißblockfläche 1103,
- 1204:: äußere Fläche, der Fläche 1203 entgegengesetzt,
- 1205:: Öffnung für die Steckhalterung 1305 bzw. 1405 der Filmklemmen 1300 bzw. 1400,
- 1205a:: vordere Öffnung auf der Fläche 1201,
- 1205b:: hintere Öffnung auf der Fläche 1202,
- 1205c:: obere Retention für die Nut 1305a,
- 1205d:: untere Retention für die Nut 1305a,

- **1300:**: **Zahnfilmklemme für die waagerechte Positionierung des Zahnfilmes, vor wiegend für Seitenzahnaufnahmen mit**
- 1301:: Rückschild,
- 1302:: Frontschild,
- 1303:: Filmauflagerille,
- 1304:: Halterung mit
- 1305:: Steckhalterung für die Öffnung 1205,
- 1305a:: Nut in der Steckhalterung 1305,
- 1305b:: Einziehung der Steckhalterung zwecks Retention in der Öffnung1205,
- 1305c und 1305d:: zweigeteilte arretierbare Steckhalterungsspitze,
- 1306:: halterungsnahe Filmklemmenkante,
- 1307:: halterungsferne Filmklemmenkante,
- 1308:: Höhe der Halterung.

- **1400:**: **Filmklemme für die senkrechte Positionierung des Zahnfilmes mit**
- 1401:: Rückschild der Filmklemme,
- 1402:: Frontschild der Filmklemme,
- 1403:: Filmauflagerille,
- 1404:: Halterung mit
- 1405:: Steckhalterung für die Öffnung 1205,
- 1406:: halterungsnahe Filmklemmenkante,
- 1407:: seitliche Fläche bezeichnend für die Höhe der Halterung.

- **1500:**: **Führungsschiene mit**
- 1501:: erhobene Markierung entlang der Längsachse der Führungsschiene.

- **1600:**: **Zentriervorrichtung,**

- **1601:**: **Indikatorstab,**
- 1601a:: innere Aussparung für die erhobene Markierung 1501 für die Positionierung der Zentriervorrichtung im dritten ( unten links) und im ersten (oben rechts) Quadranten,
- 1601b:: innere Aussparung für die erhobene Markierung 1501 für die Positionierung der Zentriervorrichtung im vierten ( unten rechts) und im zweiten ( oben links) Quadranten,

- **1602:**: **Verbindung zwischen dem Indikatorstab und dem Visierring,**

- **1603:**: **Visierring mit**
- 1604:: erhobene Markierung für die senkrechte oder waagerechte Positionierung der Aussparung 1701 der Rechteckblende 1700,
- 1605:: eckige Markierung am Visierring für die Positionierung eines rechteckigen Langtubus,
- 1606:: runde Markierung für die Positionierung des Röntgenlangtubus,
- 1607:: äußerer tubusnaher Ring,
- 1608:: innerer tubusferner Ring,

- **1700:**: **Rechteckblende mit**
- 1701:: rechteckige Aussparung,
- 1702:: kurzer Schenkel der rechteckigen Aussparung,
- 1703:: langer Schenkel der rechteckigen Aussparung,
- 1704:: randständige Aussparung entsprechend der erhobenen Markierung 1604 in der Höhe und mittig des langen Schenkels 1703,
- 1705:: randständige Aussparung entsprechend der erhobenen Markierung 1604 in der Höhe und mittig des kurzen Schenkels 1702.

## Patentansprüche

1. Filmhalter für die Anfertigung von Meßaufnahmen mit der Paralleltechnik bei angelegtem Kofferdamrahmen,
mit einem Aufbißblock,
mit einer Filmklemme,
mit einer den Aufbißblock und die Filmklemme verbindenden Verbindung, **dadurch gekennzeichnet, daß** die Verbindung lösbar ist.

2. Filmhalter nach Anspruch 1,
wobei die Verbindung ein Verbindungsstück zum Verbinden von Aufbißblock und Filmklemme aufweist.

3. Filmhalter nach Anspruch 2,
wobei das Verbindungsstück mindestens an dem Aufbißblock lösbar befestigt ist.

4. Filmhalter nach Anspruch 3,
wobei das Verbindungsstück mittels einer Steckverbindung an dem Aufbißblock lösbar befestigt ist.

5. Filmhalter nach Anspruch 4,
wobei die Steckverbindung durch mindestens einen Zapfen an dem Verbindungsstück und einer entsprechenden Aufnahme in dem Aufbißblock herstellbar ist.

6. Filmhalter nach Anspruch 5,
wobei mindestens zwei runde, im wesentlichen parallel angeordnete Zapfen vorgesehen sind, die zur Herstellung der Steckverbindung mit entsprechenden, die Aufnahme für die Zapfen bildenden Bohrungen zusammenwirken.

7. Filmhalter nach einem der Ansprüche 2 bis 6,
wobei das Verbindungsstück mindestens an der Filmklemme lösbar befestigt ist.

8. Filmhalter für die Anfertigung von Röntgenaufnahmen bei angelegtem Kofferdamrahmen, insbesondere nach einem der vorstehenden Ansprüche,
mit einem Aufbißblock,
mit einer Filmklemme,
mit einer den Aufbißblock und die Filmklemme verbindenden Verbindung, **dadurch gekennzeichnet, daß** die Verbindung derart ist, daß der Aufbißblock gegenüber einer auf der Ebene der Filmklemme stehenden Normalen, bevorzugt um mindestens eine Zahnbreite, versetzt angeordnet ist.

9. Filmhalter nach Anspruch 8,
wobei die Verbindung ein Verbindungsstück zum Verbinden von Aufbißblock und Filmklemme aufweist,
und wobei das Verbindungsstück das mesiale und distale Ende des Aufbißblockes mit der Filmklemme, bevorzugt im wesentlichen im rechten Winkel zur Längsachse des Aufbißblockes, weiter bevorzugt im wesentlichen parallel zur Ebene der Filmklemme, verbindet.

10. Filmklemme nach einem der Ansprüche 7 bis 9,
wobei das Verbindungsstück an seinem dem Aufbißblock entgegengesetztem Ende eine Aufnahme für die Filmklemme aufweist, welche Aufnahme eine Drehung im wesentlichen um die auf der Ebene der Filmklemme stehende Normale, weiter bevorzugt auch eine lösbare Befestigung der Filmklemme an dem Verbindungsstück erlaubend, ermöglicht.

11. Filmklemmen nach einem der Ansprüche 7 bis 10,
wobei an einem der Filmklemme abgewandten Ende des Filmhalters ein Visierring im wesentlichen mit der Filmklemme fluchtend angeordnet ist.

12. Filmklemme nach Anspruch 11,
wobei der Visierring im wesentlichen auf eine auf der Mitte der Filmklemme auf der Ebene der Filmklemme stehende Normale zentrierbar ist, so dass eine Visierlinie zwischen Visierring und Filmklemme gegenüber dem Aufbißblock versetzt verläuft.
